Europäisches Patentamt

European Patent Office

Office européen des brevets

(11) Publication number: **0 144 476**
**B1**

(12) **EUROPEAN PATENT SPECIFICATION**

(45) Date of publication of patent specification: **07.10.87**

(21) Application number: **83307558.3**

(22) Date of filing: **13.12.83**

(51) Int. Cl.⁴: **C 07 C 15/46,** C 07 C 5/333, C 07 C 5/48

(54) Dehydrogenation of dehydrogenatable hydrocarbons.

(43) Date of publication of application:
**19.06.85 Bulletin 85/25**

(45) Publication of the grant of the patent:
**07.10.87 Bulletin 87/41**

(84) Designated Contracting States:
**BE CH DE FR GB IT LI NL SE**

(56) References cited:
**US-A-3 409 688**
**US-A-3 437 703**
**US-A-3 439 058**
**US-A-3 670 044**
**US-A-3 855 330**

(73) Proprietor: **UOP Inc.**
**10 UOP Plaza Algonquin & Mt. Prospect Roads**
**Des Plaines Illinois 60016 (US)**

(72) Inventor: **Imai, Tamotsu**
**509, North Eastwood Avenue**
**Mount Prospect Illinois 60056 (US)**

(74) Representative: **Newby, Martin John et al**
**J.Y. & G.W. Johnson Furnival House 14-18 High**
**Holborn**
**London WC1V 6DE (GB)**

Courier Press, Leamington Spa, England.

## Description

It has been known in the prior art that unsaturated hydrocarbons may be obtained from the dehydrogenation of dehydrogenatable hydrocarbons. The dehydrogenation may be effected by subjecting the dehydrogenatable hydrocarbons to a dehydrogenation process at dehydrogenation conditions in the presence of certain catalytic compositions of matter which possess the ability to dehydrogenate said compounds with the resultant formation of olefinic hydrocarbons. The particular dehydrogenation catalysts which are employed are well known in the art and comprise such compounds as nickel composited on a solid support such as diatomaceous earth, kieselguhr or charcoal and iron composited on the same supports.

Other dehydrogenation processes have employed, in addition to the dehydrogenation catalyst, an oxidation catalyst in the reaction process. The presence of the oxidation catalyst is necessitated by the fact that it is advantageous to oxidize the hydrogen produced, by contact with an oxygen-containing gas, in order to maintain the desired reaction temperature. For example, styrene, which is an important chemical compound utilized for the preparation of polystyrene, plastics, resins or synthetic elastomers such as styrene-butadiene rubber, etc., may be prepared from the dehydrogenation of ethylbenzene. The dehydrogenation of ethylbenzene to styrene, which is effected by treating ethylbenzene with steam in the presence of a modified iron catalyst, is endothermic in nature. The heat of reaction is about 30 Kcal per mole of ethylbenzene. Therefore, the temperature of the catalyst bed decreases significantly during the progress of the reaction in a commercial adiabatic reactor, resulting in limitation of ethylbenzene conversion to a low level. The limitation of conversion arises from the fact that the equilibrium conversion of ethylbenzene is lowered and the rate of ethylbenzene dehydrogenation decreases as the reaction temperature decreases. The decrease of temperature adversely affects not only the conversion level, but also the selectivity for styrene, since, at equilibrium conditions, only undesirable side reactions continue to take place. Therefore, it is necessary to maintain the desired temperature level in order to provide a high equilibrium conversion level and a high reaction rate. In the conventional process, the maintenance of temperature is attained by reheating the product stream with the addition of superheated steam between dehydrogenation catalyst beds using a multi-catalyst bed reactor system. However, consumption of the additional superheated steam is considerably high and makes the dehydrogenation process costly. Accordingly, significant process economic improvements over the conventional ethylbenzene dehydrogenation processes can be achieved if the reaction temperature is somehow maintained while eliminating or reducing the additional superheated steam. One method of providing for the maintenance of the reaction temperature is to introduce oxygen into the reaction mixture by way of oxygen or an oxygen-containing gas such as air which will burn the hydrogen formed during the dehydrogenation reaction, this combustion resulting in an exothermic reaction which will provide the necessary amount of heat and, in addition, will shift the equilibrium toward production of styrene since the hydrogen formed in the dehydrogenation is consumed. Consequently, a higher conversion and higher styrene selectivity are achievable.

The combustion of hydrogen with the oxygen in the oxygen-containing gas requires the presence of an oxidation catalyst. There are some key requirements for the oxidation catalyst to be usable for such a purpose. The most important catalytic property required is good catalytic stability since the oxidation catalyst must survive under very severe reaction conditions, namely at about 600° to 650°C in the presence of steam. Under such conditions, porous inorganic materials such as aluminas, silicas and zeolites cannot maintain their pore structures for a long period of time, resulting in the permanent damage of catalysts prepared using such materials as supports, e.g. platinum supported on a porous high surface area alumina, silica, or zeolite. Secondly, the oxidation catalyst must be very active to achieve complete conversion of oxygen to avoid poisoning of iron-based dehydrogenation catalysts which are sensitively oxidized with oxygen to lose their dehydrogenation activities. Thirdly, the oxidation catalyst must be selective for oxidation of hydrogen. Otherwise, ethylbenzene and styrene are consumed to lower the efficiency of styrene production.

Various U.S. patents have described types of oxidation catalysts which may be employed in this process. For example, US—A—3,437,703 describes a catalytic dehydrogenation process which employs, as a dehydrogenation catalyst, an alkaline metal-promoted iron compound, namely a composition known in the trade as Shell-105 which consists of from 87% to 90% ferric oxide, 2% to 3% chromium oxide, and from 8% to 10% of potassium oxide. In addition, another dehydrogenation catalyst which is employed comprises a mixture of nickel, calcium, chromic oxide, graphite with a major portion of a phosphate radical. In addition to these dehydrogenation catalysts, the reaction also employs a catalyst for the oxidation step of the process comprising platinum or palladium in elemental form or as a soluble salt. US—A—3,380,931 also discloses an oxidation catalyst which may be used in the oxidative dehydrogenation of compounds such as ethylbenzene to form styrene comprising an oxide of bismuth and an oxide of a metal of Group VIB of the Periodic Table such as molybdenum oxide, tungsten oxide or chromium oxide. In addition, the patent also states that minor amounts of arsenic may also be present in the catalytic composite as well as other metals or metalloids such as lead, silver, tin, manganese, phosphorus, silicon, boron and sulfur.

US—A—3,855,330 discloses a method for the production of styrene in which ethylbenzene is treated in the vapor state by successive passage over a dehydrogenation catalyst in the presence of steam and an oxidation catalyst, an oxygen-containing gas being introduced into the reaction medium for contact with the oxidation catalyst. The dehydrogenation catalysts which are employed are those which have been set forth in various prior U.S. patents and which may be similar in nature to the dehydrogenation catalysts previously discussed. Amongst those are alkaline-metal-promoted iron compounds. The oxidation catalysts employed included noble metals of Group VIII of the Periodic Table, especially platinum or palladium composited on inorganic supports such as alumina or molecular screens of the zeolite type which have been charged with ferrous, heavy or noble metals. The patent lists catalysts which can be employed, including copper or various zeolites, platinum on alumina, platinum on spinel, platinum and sodium on zeolites, and platinum, sodium and potassium on zeolites.

US—A—3,670,044 discloses a method for dehydrogenating cycloalkane, arylalkane and alkanes in the presence of gaseous hydrogen or mixture of gaseous hydrogen and gaseous oxygen using a catalyst composition comprising a Group VIII metal or a mixture of a Group VIII metal and a Group IVA metal deposited on a support comprising a Group II aluminate spinel. It is noted that the patentee teaches that added hydrogen is used in connection with the oxygen, and that when only oxygen is used, the conversion and selectivity are generally low. The addition of hydrogen is believed to be a significant disadvantage in the dehydrogenation processs inasmuch as the equilibrium conversion is lowered. This is in contradistinction to the process of the present invention wherein the dehydrogenation process, prior to the oxidation step, is not effected in the presence of any added hydrogen. As will hereinafter be shown in greater detail, the present process results in the selective oxidation of hydrogen with a concomitantly lower selectivity to carbon monoxide and carbon dioxide. In addition, the patentee teaches the use of one catalyst for both dehydrogenation and oxidation which is in contrast to the separate dehydrogenation and oxidation catalysts which are used in the present process.

In a process for the dehydrogenation of dehydrogenatable hydrocarbons wherein said hydrocarbons are treated with steam and a dehydrogenation catalyst along with a subsequent or concurrent treatment with an oxygen-containing gas in the presence of an oxidation catalyst, it will hereinafter be shown that by utilizing a particular class of dehydrogenation catalysts it is possible to obtain the desired product in an excellent yield with a concomitant use of the catalyst for a longer period of time due to the excellent stability of the catalyst during the reaction period.

According to the present invention a process for the dehydrogenation of a dehydrogenatable hydrocarbon comprises contacting said hydrocarbon with a dehydrogenation catalyst comprising an alkaline-promoted iron compound at dehydrogenation conditions in the presence of steam, thereafter contacting the resultant mixture of unconverted hydrocarbon, dehydrogenated hydrocarbon, hydrogen and steam with an oxygen-containing gas at oxidation conditions in the presence of an oxidation catalyst comprising a noble metal of Group VIII and a metal of Group IVA of the Periodic Table composited on a highly porous inorganic support, thereby oxidizing hydrogen contained in said mixture to reheat said mixture, and contacting the thus reheated mixture with additional dehydrogenation catalyst comprising an alkaline-metal-promoted iron compound at dehydrogenation conditions. By utilizing the particular selective oxidation catalyst, it is possible selectively to oxidize the hydrogen which is present with minimum oxidation of the hydrocarbons and thereby to obtain dehydrogenated hydrocarbon in a relatively high yield as well as to maintain the stability and activity of the catalyst. This obviates the necessity for regenerating or changing the catalyst, and thus adds to the economic feasibility of the dehydrogenation process.

A specific embodiment of this invention is found in a process for the dehydrogenation of ethylbenzene which comprises contacting said ethylbenzene with a dehydrogenation catalyst comprising a modified iron catalyst at a temperature in the range of from 500° to 700°C and a pressure in the range of from 0.1 to 10 atm. (10 to 1000 kPa) in the presence of steam, thereafter contacting the resultant mixture of unconverted ethylbenzene, styrene, hydrogen and steam with air in the presence of a catalyst comprising a mixture of platinum, tin, and potassium composited on alumina at a temperature in the range of from 500° to 700°C and a pressure in the range of from 0.1 to 10 atm (10 to 1000 kPa), whereby hydrogen is selectively oxidized, and recovering styrene.

As hereinbefore set forth, the present invention is concerned with a dehydrogenation process which involves the use, in one step of the process, of a selective oxidation catalyst. In the present process, a dehydrogenatable hydrocarbon of the type hereinafter set forth in greater detail, is contacted with a dehydrogenation catalyst in the presence of steam in a multi-catalyst bed system. Inasmuch as the dehydrogenation of the hydrocarbon is endothermic in nature, it is necessary to provide an additional amount of heat before the product enters the next catalyst bed, the reaction temperature being held at a relatively high rate in order to provide a high equilibrium conversion as well as a high reaction rate. This increase in the desired temperature is achieved by providing an internal catalytic combustion of the hydrogen produced during the dehydrogenation reaction in order to reheat the product to the desired level. By effecting a selective oxidation of the hydrogen, it is possible to avoid the use of superheated steam or other outside sources of heat. This selective oxidation of hydrogen with the resultant composition thereof is effected by utilizing a selective oxidation catalyst of the type hereinafter set forth in greater detail the selective oxidation catalyst maintaining its stability and activity for a considerable length of time.

The process of the present invention may be effected by utilizing an apparatus in which the dehydrogenation catalyst and the oxidative catalyst, both of the type hereinafter set forth in greater detail, are loaded in the apparatus in alternate layers. The number of alternate layers of dehydrogenation catalyst and selective oxidative catalyst may vary according to the size or type of apparatus which is employed, the number of alternate layers ranging from three to about nine. As will hereinafter be shown, the dehydrogenation catalyst and the oxidation catalyst are different in nature. The dehydrogenation catalysts employed comprise an alkaline-metal-promoted iron compound. The term "alkaline metal" as used in the present specification refers to metals of Groups IA and IIA of the Periodic Table, which includes lithium, sodium, potassium, rubidium, cesium, beryllium, magnesium, calcium, strontium and barium. In addition, the promoted iron compound catalyst will, in preferred embodiments of the invention, also include a compound containing a metal of Group IVB, VB or VIB of the Periodic Table. For example, a typical dehydrogenation catalyst which may be employed in the process of this invention consists essentially of about 85% by weight of ferric oxide, 12% by weight of potassium hydroxide, 2% by weight of chromia and 1% by weight of sodium hydroxide. Another typical dehydroganation catalyst which may be used comprises 90% by weight of ferric oxide, 4% by weight of chromia and 6% by weight of potassium carbonate. In addition to these catalysts, other well-known dehydrogenation catalysts which may be utilized include those comprising ferric oxide, potassium oxide, as well as other metal oxides and/or sulfides of metals of Groups IA, IIA, IVB, VB and VIB of the Periodic Table including those of calcium, lithium, strontium, magnesium, beryllium, zirconium, tungsten, molybdenum, hafnium, vanadium, copper, chromium and mixtures of two or more oxides such as chromia-alumina, chromia-titania, alumina-vanadia and the like.

The dehydrogenation of a dehydrogenatable hydrocarbon such as, for example, ethylbenzene, is effected by contacting the dehydrogenatable hydrocarbon and steam, in the absence of any added hydrogen, with the aforesaid catalyst at dehydrogenation conditions. These conditions are generally temperatures in the range of from 500° to 700°C and at reaction pressures in the range of from 0.1 to 10 atm (10 to 1000 kPa); the exact dehydrogenation conditions are, however, a function of the particular dehydrogenatable hydrocarbon undergoing dehydrogenation. Other reaction conditions typically include a Liquid Hourly Space Velocity based on the hydrocarbon charge of from 0.1 to 10 hrs$^{-1}$ and a steam to hydrocarbon weight ratio of from 1:1 to 40:1. The number of dehydrogenation zones of the catalyst beds may vary from 1 to about 5 in number and typically may comprise three reaction zones; however, the number of zones is not critical to the invention. After contacting the dehydrogenation catalyst with the steam and hydrocarbon the resulting mixture comprising unconverted hydrocarbon, dehydrogenated hydrocarbon, steam and hydrogen which has passed through the catalyst bed is contacted in a separate zone with the selective oxidative catalytic composition of the type hereinafter set forth in greater detail. In addition, oxygen-containing gas is introduced into the reactor, preferably at a point adjacent to the oxidative catalyst bed. Examples of oxygen-containing gases which may be utilized to effect the selective oxidation of the hydrogen present will include air, oxygen, air or oxygen diluted with other gases such as steam, carbon dioxide and inert gases such as nitrogen, argon and helium. The amount of oxygen which is introduced to contact the product stream may range from 0.1:1 to 2:1 moles of oxygen per mole of hydrogen contained in the product stream. In this particular reaction zone, the product stream, which comprises unreacted dehydrogenatable hydrocarbon, dehydrogenated hydrocarbon, hydrogen and steam, undergoes a selective oxidation in contact with oxygen and the oxidative catalyst whereby hydrogen is selectively oxidized to water with a minimal amount of reaction of oxygen with the hydrocarbons, either unconverted hydrocarbon or dehydrogenated hydrocarbon.

After passage through the zone containing the oxidation catalyst, the mixture may then be passed through a second dehydrogenation zone containing a dehydrogenation catalyst of the type hereinbefore set forth for further dehydrogenation, the process being completed through the plurality of zones followed by withdrawal of the product stream and separation of the unconverted hydrocarbon from the desired dehydrogenated product.

It is contemplated that the dehydrogenation process for the dehydrogenation of dehydrogenatable hydrocarbons utilizing the oxidative catalytic compositions of matter of the present invention will be applicable to a wide variety of dehydrogenatable hydrocarbons. Examples of hydrocarbons which are susceptible to a dehydrogenation process utilizing the catalysts of the present invention include lower alkyl-substituted aromatic hydrocarbons such as ethylbenzene, diethylbenzene, isopropylbenzene, diisopropylbenzene, o-ethyltoluene, m-ethyltoluene, p-ethyltoluene, o-isopropyltoluene, m-isopropyltoluene, p-isopropyltoluene, ethylnaphthalene, propylnaphthalene, isopropylnaphthalene and diethylnaphthalene, paraffins such as ethane, propane, n-butane, isobutane, n-pentane, isopentane, n-hexane, n-heptane, n-octane, n-nonane, n-decane and branched chain isomers thereof, cycloparaffins such as cyclobutane, cyclopentane, cyclohexane, methylcyclopentane, methylcyclohexane and ethylcyclopentane, and olefins such as 1-butene, 2-butene, 1-pentene, 2-pentene, 1-hexene, 2-hexene, 3-hexene and branched chain derivatives thereof.

The selective oxidation catalysts employed in the process of the present invention comprise a noble metal of Group VIII of the Periodic Table and a metal of Group IVA of the Periodic Table composited on a highly porous inorganic support, and preferably an inorganic highly porous material possessing a relatively high surface area, suitably a surface area within the range of from 1 to 500 m$^2$/g. In addition, if so desired, it is also contemplated within the scope of this invention that the catalyst composite will also contain a metal selected from Groups IA and IIA of the Periodic Table. Of the noble metals of Group VIII of the Periodic Table, platinum, palladium and rhodium comprise the preferred species, said metals suitably being present in the final composite in an amount in the range of from 0.01% to 5% by weight. As the metal of Group IVA of the Periodic Table, germanium, tin and lead are preferably present in the final catalyst composite in an amount in the range of from 0.01% to 5% by weight, tin being particularly preferred. The preferred species of metals of Group IA or IIA of the Periodic Table are potassium, rubidium, cesium, barium, and strontium, the alkali metals or alkaline earth metals preferably being present in an amount in the range of from 0.01% to 10% by weight of the catalyst composite. The aforesaid metals are composited on a solid inorganic support having the necessary highly porous configuration. Some specific examples of these highly porous supports which may be employed include aluminas such as alpha-alumina, gamma-alumina, eta-alumina and theta-alumina, silica, mixtures of inorganic oxides such as silica-alumina, silica-zirconia, alumina-zirconia-silica, and silicon carbide. The selective oxidation catalyst which is utilized in the process of this invention may be prepared in any suitable manner known in the art. For example, one type of preparation will comprise impregnating the solid support which may for example be in the form of beads, spheres or pellets with an aqueous solution of a Group VIII noble metal compound of the Periodic Table. The aqueous solution of the noble metal-containing compound may be prepared from soluble salts of these metals, such as chloroplatinic acid, chloropalladic acid, rhodium chloride, platinum sulfate or palladium sulfate. The solid support is impregnated with the solution for a period of time which is sufficient to allow the deposition of the desired amount of the noble metal on the solid support, preferably an amount sufficient so that the finished catalytic composition will contain from 0.01% to 5% by weight of the composite. After recovery of the impregnated solid support, the composite is then dried and calcined at a temperature in the range of from 500° to 600°C or more in an air atmosphere.

The thus formed composite containing a noble metal may then be further impregnated with an aqueous solution of a metal of Group IVA of the Periodic Table. In a similar manner to that hereinbefore described, the amount of a soluble salt such as tin chloride, tin bromide, tin sulfate, lead chloride, lead persulfate or germanium chloride present in the solution will be sufficient so that the finished catalytic composition will contain the desired amount of Group IVA metal. Again, the impregnation is allowed to proceed for a predetermined period of time following which the composite is recovered, dried and calcined. In the event that it is desired to have a metal of Group IA or IIA of the Periodic Table present in the catalyst composite, the third step of the process is effected in a similar manner by subjecting the composite to an impregnation utilizing an aqueous solution containing the desired metal. Examples of salts of these metals which may be employed include potassium chloride, potassium bromide, potassium iodide, potassium nitrate, potassium sulfate, potassium acetate, potassium propionate, rubidium chloride, rubidium bromide, rubidium iodide, rubidium nitrate, rubidium sulfate, rubidium acetate, rubidium propionate, cesium chloride, cesium bromide, cesium iodide, cesium nitrate, cesium sulfate, cesium acetate, cesium propionate, calcium chloride, barium chloride, barium bromide, barium iodide, barium nitrate, barium sulfate, barium acetate and barium propionate. After allowing the impregnation to proceed for a period of time sufficient to permit the deposition of the desired amount of metal on the catalyst, the composite is recovered, dried and calcined at a temperature within the range hereinbefore set forth, and recovered.

It is also contemplated that the selective oxidation catalyst may be prepared by co-impregnating the noble metal of Group VIII of the Periodic Table, the metal of Group IVA of the Periodic Table, and, if so desired, the metal of Group IA or IIA of the Periodic Table on the solid support. When such a type of preparation is employed, the solid support, such as alumina, is impregnated with an aqueous solution containing salts of the noble metal and the Group IVA metal along with, if so desired, the alkali metal or alkaline earth metal in a manner similar to that hereinbefore set forth. After allowing the impregnation to proceed for a predetermined period of time, the composite is recovered, dried and calcined at a temperature within the range hereinbefore set forth in an air atmosphere, following which it is recovered for use in the dehydrogenation process of the present invention.

Some specific examples of selective oxidation catalytic compositions of matter which may be used in the process of the present invention include platinum, germanium and potassium composited on alumina, palladium, germanium and potassium composited on alumina, rhodium, germanium and potassium composited on alumina, platinum, tin and potassium composited on alumina, palladium, tin and potassium composited on alumina, rhodium, tin and potassium composited on alumina, platinum, germanium and cesium composited on alumina, palladium, germanium and cesium composited on alumina, rhodium, germanium and cesium composited on alumina, platinum, tin and cesium composited on alumina, palladium, tin and cesium composited on alumina, rhodium, tin and cesium composited on alumina, platinum, germanium and barium composited on alumina, palladium, germanium and barium composited on alumina, rhodium, germanium and barium composited on alumina, platinum, tin and barium composited on alumina, palladium, tin and barium composited on alumina, rhodium, tin and barium composited on alumina, platinum, lead and potassium composited on alumina, palladium, lead and potassium composited on alumina, and rhodium, lead and potassium composited on alumina. It is to be understood that the above enumerated catalysts are only representative of the selective oxidation composites which may be used in the process of this invention, and that said invention is not necessarily limited thereto. By utilizing a selective oxidative catalytic composition of matter in a process which involves the dehydrogenation of dehydrogenatable hydrocarbons, it is possible to obtain a process which, in addition to obtaining a desirable and commercially attractive yield of dehydrogenation products, also permits the operation of the process in an economically viable manner due to the catalytic stability of the catalyst under the relatively harsh and stringent operating conditions such as high temperature and high concentration of steam at which the process is operated. This is in contradistinction to prior art types of oxidative catalysts which do not possess the stability of the present catalysts and cannot survive for a long period of time, thus making the commercial use of such catalysts unattractive due to the necessity of having to replace or regenerate the catalyst after a short interval of operating time has elapsed. In addition, the catalysts of the present invention also exhibit a definite affinity for the selective oxidation of hydrogen rather than a tendency for the oxidation of the dehydrogenated products.

The following examples are given for purposes of illustrating the process of the present invention utilizing a selective oxidation catalyst in said process. However, it is to be understood that these examples are given merely for purposes of illustration and that the present invention is not limited thereto.

Example I (Comparative)

In this example, an oxidation catalyst was prepared by a procedure in which the first step involved impregnating 500 cc of 1.6 mm (1/16″) diameter gamma-alumina beads with an aqueous chloroplatinic acid solution at about 100°C. The alumina was impregnated for a period of 2 hours, following which the impregnated alumina was dried and calcined at a temperature of about 540°C for a period of 2 hours in the presence of a gas consisting of a mixture of air and steam. The impregnated sample was then further impregnated with an aqueous solution of potassium nitrate at about 100°C for 2 hours following which the sample was recovered, dried and calcined at 540°C for 2 hours. The impregnated composite which was recovered from the calcination step contained 0.79% by weight of platinum and 2.78% by weight of potassium. In addition, the composite had a surface area of 152 m²/g, a pore volume of 0.41 cc/g, and an ABD of 0.522 g/cc.

The catalyst was loaded into 22.2 mm (7/8″) inner diameter stainless steel tube reactor having a 254 mm (10″) long 12.7 mm (1/2″) diameter bore for the catalyst loading. The reactor was heated to an inlet temperature of 600°C and a feed stream comprising a mixture of ethylbenzene, styrene, steam, hydrogen, oxygen and nitrogen which simulated a product stream at about a 60% ethylbenzene conversion from the second dehydrogenation catalyst bed of a 3-dehydrogenation catalyst bed reactor system having an oxidation catalyst bed positioned between the dehydrogenation catalyst beds, was fed to the reactor. The feed stream was passed over the oxidation catalyst bed at the aforesaid inlet temperature, and a pressure of 0.5 atm (50 kPa) at a Liquid Hourly Space Velocity of 100 hrs⁻¹. The molar feed ratio of the feed stream of ethylebenzene/styrene/steam/hydrogen/oxygen/nitrogen was 1/1.48/17.9/1.14/0.25/2.21. The conversion of the oxygen was plotted for a period of 250 hours, the results of said run being set forth in Table I below. In this Table, column A is the % conversion of oxygen and column B is the mole % selectivity for oxygen reacting to form carbon dioxide and carbon monoxide.

TABLE I

| Hours of stream | A | B |
| --- | --- | --- |
| 50 | 65 | 26 |
| 100 | 65 | 23 |
| 150 | 66 | 24 |
| 200 | 63 | 23 |
| 250 | 59 | 21 |

Example II (Catalyst required according to the invention)

A selective oxidation catalyst was prepared in a manner similar to that set forth in Example I above. In this example, 4630 cc of 1.6 mm (1/16″) diameter beads of a tin oxide-gamma-alumina cogel were impregnated with an aqueous solution of chloroplatinic acid at about 100°C. The tin oxide-alumina beads were impregnated for a period of 10 hours, following which the impregnated beads were recovered, dried and calcined at a temperature of about 320°C for a period of 30 minutes and at 565°C for 1 hour, said calcination being effected in the presence of a gas consisting of a mixture of air and steam. The sample was then impregnated with an aqueous solution of potassium nitrate at about 100°C for a period of 10 hours, following which the impregnated beads were recovered, dried and calcined at a temperature of about 320°C for 30 minutes, and at 565°C for 1 hour. The catalyst was reduced with hydrogen at about 475°C for 4 hours. The impregnated composite which was recovered from the calcination step contained 0.75% by weight of platinum, 0.5% by weight of tin, and 2.44% by weight of potassium. The composite had a surface area of 182 m²/g, a pore volume of 0.60 cc/g, and an ABD of 5.90 g/cc.

The catalyst was loaded into a 22.2 mm (7/8″) inner diameter stainless steel tube reactor having a 254

# 0 144 476

mm (10″) long 12.7 mm (1/2″) diameter bore for the catalyst loading. A feed stream comprising a mixture of ethylbenzene, styrene, steam, hydrogen, oxygen and nitrogen in a molar feed ratio similar to that set forth in Example I above, and which simulated a product stream from a dehydrogenation step was passed over the selective oxidation bed in an inlet temperature of 600°C, a pressure of 0.5 atm (50 kPa) at a Liquid Hourly Space Velocity of 100 hrs.$^{-1}$. As in Example I above, the conversion of the oxygen was plotted for a period of 250 hours. The results of this run are set forth in Table II below in which column A refers to the % conversion of oxygen and column B refers to the mole % selectivity for oxygen reacting to carbon dioxide and carbon monoxide.

TABLE II

| Hours on stream | A | B |
| --- | --- | --- |
| 50 | 62 | 10 |
| 100 | 61 | 11 |
| 150 | 59 | 8 |
| 200 | 59 | 8 |
| 250 | 59 | 9 |

It is apparent from a comparison of Tables I and II that the selective oxidation catalyst which comprised a noble metal of Group VIII of the Periodic Table, namely platinum, a metal of Group IVA of the Periodic Table, namely tin, and a metal of Group IA of the Periodic Table, namely potassium, exhibited a much lower selectivity of oxygen which reacted to form carbon dioxide and carbon monoxide than did the catalyst which contained only platinum and potassium. This is indicative of an improved selectivity to a hydrogen combustion with a concomitant low selectivity to a reaction with the hydrocarbons present, thus permitting or resulting in a greater yield of the desired dehydrogenated hydrocarbon and unconverted hydrocarbons which may be recycled back for further dehydrogenation.

Example III

To illustrate further the efficiency of the process of the present invention, a comparison is made between the instant process and the process described in US—A—3,670,044. It is noted from Example II above that for 100 hours of operation there has been a 61% conversion of the oxygen with an 11% selectivity for the oxygen reacting with ethylbenzene and styrene to form carbon dioxide. Using a feed of ethylbenzene/styrene/oxygen which had 1 mole of ethylbenzene, 1.48 moles of styrene, and 0.25 mole of oxygen, the amount of carbon dioxide which was produced equaled 0.0168 mole. Theoretically, by means of oxidation, 8 moles of carbon dioxide are produced from 1 mole of ethylbenzene or styrene, so that the amount of ethylbenzene plus styrene which has been converted into carbon dioxide is 0.0021 mole out of a total of 2.48 moles. The loss of feed to carbon dioxide due to the selective oxidation of hydrogen in the process of the present invention thus equals 0.085%.

In contradistinction to this, the patent shows, as exemplified by Example III therein, that there was a 49% conversion of $n$-butane with a 1% selectivity to carbon dioxide. Therefore, 0.49% of the $n$-butane which was fed into the system was converted into carbon dioxide. A comparison of the two figures shows therefore that the loss of feed to carbon dioxide in the patent was greater than the loss of feed to carbon dioxide in the present invention. This difference is due to the use of a selective oxidation catalyst of the type hereinbefore described.

Example IV

A comparison of the process of the present invention in which the selective oxidation of hydrogen is utilized to reheat the dehydrogenation step of the process with a process in which the dehydrogenation temperature is attained by heating with steam results in not only an advantage in the yield of the desired product, but also an advantage in the utility or energy requirements. In a conventional process in which a dehydrogenatable hydrocarbon such as ethylbenzene is treated with a dehydrogenation catalyst in the presence of steam, the results show a 70% conversion of the ethylbenzene while utilizing 23 moles of superheated steam to produce 1 mole of styrene.

Example V

In a like manner, other selective oxidation catalysts comprising platinum or rhodium along with germanium or lead and, if so desired, cesium, strontium or barium, composited on other inorganic supports such as silica, nonacidic silica-alumina, or silicon carbide which possesses physical characteristics of surface area, pore volume, and ABD's similar to that of alumina may also show similar results when used as selective oxidation catalysts in a dehydrogenation reaction involving the treatment of a dehydrogenatable hydrocarbon with a dehydrogenation catalyst.

**Claims**

1. A process for the dehydrogenation of a dehydrogenatable hydrocarbon comprising
(a) contacting the hydrocarbon with a dehydrogenation catalyst comprising an alkaline-metal-promoted iron compound at dehydrogenation conditions in the presence of steam;
(b) thereafter contacting the resulting mixture of unconverted hydrocarbon, dehydrogenated hydrocarbon, hydrogen and steam with an oxygen-containing gas at oxidation conditions in the presence of an oxidation catalyst comprising a noble metal of Group VIII of the Periodic Table composited on an inorganic support, thereby oxidising hydrogen contained in the mixture to reheat the mixture, and
(c) contacting the thus reheated mixture with additional dehydrogenation catalyst comprising an alkaline-metal-promoted iron compound at dehydrogenation conditions,
characterised in that the oxidation catalyst comprises a metal of Group IVA and a noble metal of Group VIII of the Periodic Table composited on a highly porous inorganic support.

2. A process as claimed in claim 1, characterised in that the oxidation catalyst contains from 0.01 to 5% by weight of germanium, tin or lead as the Group IVA metal component.

3. A process as claimed in claim 1 or 2, characterised in that the oxidation catalyst contains tin as the Group IVA metal component.

4. A process as claimed in any of claims 1 to 3, characterised in that the oxidation catalyst also contains from 0.01 to 10% by weight of a metal of a metal of Group IA or IIA of the Periodic Table.

5. A process as claimed in claim 4, characterised in that the metal of Group IA or IIA of the Periodic Table is selected from potassium, rubidium, cesium, strontium and barium.

6. A process as claimed in any of claims 1 to 5, characterised in that the inorganic support of the oxidation catalyst possesses a surface area in the range from 1 to 500 m$^2$/g.

7. A process as claimed in claim 6, characterised in that the inorganic support of the oxidation catalyst is selected from alumina, silica, silica-alumina and silicon carbide and the Group VIII metal component is selected from platinum, palladium and rhodium and is present in an amount of from 0.01 to 5% by weight.

8. A process as claimed in any of claims 1 to 7, characterised in that the dehydrogenatable hydrocarbon is ethylbenzene, the dehydrogenated hydrocarbon is styrene and the oxygen-containing gas is oxygen or air.

9. A process as claimed in claim 8, characterised in that the dehydrogenation conditions and the oxidation conditions include a temperature in the range from 500 to 700°C and a pressure in the range from 10 to 1000 kPa (0.1 to 10 atmospheres).

10. A process as claimed in claim 8 or 9, characterised in that the dehydrogenation catalyst contains a metal of Group IA or IIA of the Periodic Table as alkaline metal and also contains an oxide or sulfide of a metal of Group IVB, VB or VIB of the Periodic Table.

**Patentansprüche**

1. Verfahren zur Dehydrierung eines dehydrierbaren Kohlenwasserstoffs, bei dem man
(a) den Kohlenwasserstoff unter dehydrierenden Bedingungen in Gegenwart von Dampf mit einem aus einer mit einem alkalischen Metall aktivierten Eisenverbindung bestehenden Dehydrierungskatalysator in Berührung bringt,
(b) anschliessend das so entstandene Gemisch aus nichtumgesetztem Kohlenwasserstoff, dehydriertem Kohyenwasserstoff, Wasserstoff und Dampf unter oxydierenden Bedingungen in Gegenwart eines aus einem auf einem anorganischen Träger abgeschiedenen Edelmetal aus Gruppe VIII des periodischen Systems bestehenden Katalysators mit einem sauerstoffhaltigen gas in Berührung bringt und dadurch den im Gemisch enthaltenen Wasserstoff zu dessen Wiederaufheizung oxydiert und
(c) das so wieder aufgeheizte Gemisch unter dehydrierenden Bedingungen mit weiterem aus einer mit einem alkalischen Metall aktivierten Eisenverbindung bestehenden Dehydrierungskatalysator in Berührung bringt, dadurch gekennzeichnet, dass der Oxydationskatalysator aus einem Metall aus Gruppe IVA und einem Edelmetall aus Gruppe VIII des periodischen Systems besteht, die auf einem Hochporösen anorganischen Träger abgeschieden sind.

2. Verfahren nach Anspruch 1, dadurch gekennzeichnet, dass der Oxydationskatalysator 0,01 bis 5 Gew.-% Germanium, Zinn oder Blei als die Metallkomponente aus Gruppe IVA enthält.

3. Verfahren nach Anspruch 1 oder 2, dadurch gekennzeichnet, dass der Oxydationskatalysator Zinn als Metallkomponente aus Gruppe IVA enthält.

4. Verfahren nach einem der Ansprüche 1 bis 3, dadurch gekennzeichnet, dass der Oxydationskatalysator ferner 0,01 bis 10 Gew.-% eines Metall aus Gruppe IA oder IIA des periodischen Systems enthält.

5. Verfahren nach Anspruch 4, dadurch gekennzeichnet, dass das Metall aus Gruppe IA und IIA des periodischen Systems unter Kalium, Rubidium, Cäsium, Strontium und Barium ausgewählt ist.

6. Verfahren nach einem der Ansprüche 1 bis 5, dadurch gekennzeichnet, dass der anorganische Träger des Oxydationskatalysators eine Oberfläche im Bereich von 1 bis 500 m²/g besitzt.

7. Verfahren nach Anspruch 6, dadurch gekennzeichnet, dass der anorganische Träge des Oxidationskatalysators unter Aluminiumoxyd, Siliciumdioxyd, Siliciumdioxyd/Aluminiumoxyd und Siliciumcarbid ausgewählt ist sowie die Metallkomponente aus Gruppe VIII unter Platin, Palladium und Rhodium ausgewählt ist und in einer Menge von 0,01 bis 5 Gew.-% vorliegt.

8. Verfahren nach einem der Ansprüche 1 bis 7, dadurch gekennzeichnet, dass der dehydrierbare Kohlenwasserstoff Äthylbenzol, der dehydrierte Kohlenwasserstoff Styrol und das sauerstoffhaltige Gas Sauerstoff oder Luft ist.

9. Verfahren nach Anspruch 8, dadurch gekennzeichnet, dass die dehydrierenden Bedingungen und oxydierenden Bedingungen unter anderem eine Temperatur im Bereich von 500 bis 700°C und einen Druck im Bereich von 10 bis 1000 kPa (0,1 bis 10 Atmosphären) darstellen.

10. Verfahren nach Anspruch 8 oder 9, dadurch gekennzeichnet, dass der Dehydrierungskatalysator als alkalisches Metall ein Metall aus Gruppe IA oder IIA des periodischen Systems und ferner ein Oxyd oder Sulfid eines Metalls aus Gruppe IVB, VB oder VIB des periodischen Systems enthält.

## Revendications

1. Un procédé pour la déshydrogénation d'un hydrocarbure déshydrogénable comprenant

(a) la mise en contact de l'hydrocarbure avec un catalyseur de déshydrogénation comprenant un composé de fer activé par un métal alcalin dans des conditions de déshydrogénation en présence de vapeur.

(b) la mise en contact ensuite du mélange résultant d'hydrocarbure non transformé, d'hydrocarbure déshydrogéné, d'hydrogène et de vapeur avec un gaz contenant de l'oxygène dans des conditions d'oxydation, en présence d'un catalyseur d'oxydation comprenant un métal noble du groupe VIII de la classification périodique mis sous forme de composite sur un support minéral, en oxydant par là l'hydrogène contenu dans le mélange pour réchauffer le mélange et,

(c) la mise en contact du mélange ainsi réchauffé avec un catalyseur de déshydrogénation supplémentaire comprenant un composé de fer activé par un métal alcalin dans des conditions de déshydrogénation,

caractérisé en ce que le catalyseur d'oxydation comprend un métal du groupe IVA et un métal noble du groupe VIII de la classification périodique mis sous forme de composite sur un support minéral fortement poreux.

2. Un procédé selon la revendication 1, caractérisé en ce que le catalyseur d'oxydation contient de 0,01 à 10% en poids de germanium, d'étain ou de plomb en tant qu'élément métallique du groupe IVA.

3. Un procédé selon la revendication 1 ou 2, caractérisé en ce que le catalyseur d'oxydation contient de l'étain en tant qu'élément métallique du groupe IVA.

4. Un procédé selon l'une quelconque des revendications 1 à 3, caractérisé en ce que le catalyseur d'oxydation contient également de 0,01 à 10% en poids d'un métal du groupe IA ou IIA de la classification périodique.

5. Un procédé selon la revendication 4, caractérisé en ce que le métal du group IA our IIA de la classification périodique est choisi parmi le potassium, le rubidium, le césium, le strontium et le baryum.

6. Un procédé selon l'une quelconque des revendications 1 à 5, caractérisé en ce que le support minéral du catalyseur d'oxydation possède une surface spécifique comprise dans l'intervalle de 1 à 500 m²/g.

7. Un procédé selon la revendication 6, caractérisé en ce que le support minéral du catalyseur d'oxydation est choisi parmi l'alumine, la silice, la silice-alumine et le carbure de silicium et l'élément métallique du groupe VIII est choisi parmi le platine, le palladium et le rhodium et est présent dans une quantité comprise entre 0,01 et 5% en poids.

8. Le procédé selon l'une quelconque des revendications 1 à 7, caractérisé en ce que l'hydrocarbure déshydrogénable est l'éthylbenzène, l'hydrocarbure déshydrogéné est le styrène et le gaz contenant de l'oxygène est l'oxygène ou l'air.

9. Un procédé selon la revendication 8, caractérisé en ce que les conditions de déshydrogénation et les conditions d'oxydation comportent une température comprise dans l'intervalle de 500 à 700°C et une pression comprise dans l'intervalle de 10 à 1000 kPa (0,1 à 10 atmosphères).

10. Un procédé selon la revendication 8 ou 9, caractérisé en ce que le catalyseur de déshydrogénation contient un métal du groupe IA ou IIA de la classification périodique en tant que métal alcalin et contient également un oxyde ou sulfure d'un métal du groupe IVB, VB ou VIB de la classification périodique.